# EUROPÄISCHE PATENTSCHRIFT

(11) **EP 1 709 010 B1**
(45) Veröffentlichungstag und Bekanntmachung des Hinweises auf die Patenterteilung: **25.07.2007**
(21) Anmeldenummer: 05700923.5
(22) Anmeldetag: 14.01.2005
(51) Int. Cl.: C07D 239/48, C07D 239/54

(54) **VERFAHREN ZUR HERSTELLUNG VON 2-AMINO-4,6-DICHLOR-5-FORMAMIDOPYRIMIDIN**
METHOD FOR PRODUCING 2-AMINO-4,6-DICHLORO-5-FORMAMIDOPYRIMIDINE
PROCEDE POUR PRODUIRE DE LA 2-AMINO-4,6-DICHLORO-5-FORMAMIDOPYRIMIDINE

(30) Priorität: 15.01.2004 DE 102004002055
(43) Veröffentlichungstag der Anmeldung: 11.10.2006
(73) Patentinhaber: Degussa AG, 40474 Düsseldorf (DE)
(72) Erfinder: GÜTHNER, Thomas, 83308 Trostberg (DE); NEUHAUSER, Karl-Heinz, 83308 Trostberg (DE)
(86) Internationale Anmeldenummer: PCT/EP2005/000325
(87) Internationale Veröffentlichungsnummer: WO 2005/068438

(56) Entgegenhaltungen:
- EP-A- 0 684 236
- US-B1- 6 552 193
- DALUGE S M ET AL: "AN EFFICIENT, SCALABLE SYNTHESIS OF THE HIV REVERSE TRANSCRIPTASE INHIBITOR ZIAGEN (1592U89)" NUCLEOSIDES, NUCLEOTIDES AND NUCLEIC ACIDS, MARCEL DEKKER, ANN HARBOR, MI, US, Bd. 19, Nr. 1/2, 2000, Seiten 297-327, XP009011555 ISSN: 1525-7770

## Beschreibung

2-Amino-4,6-dichlor-5-formamidopyrimidin stellt ein wertvolles Zwischenprodukt für die Herstellung von Purinderivaten dar, wie sie z.B. als pharmazeutische Wirkstoffe bei der Behandlung von viralen Erkrankungen, z.B. bei der Bekämpfung von AIDS, Anwendung finden. Ein derartiges Medikament sowie Wege zu dessen Herstellung wurde ausführlich beschrieben (vgl. Susan M. Daluge et. al., Nucleosides, Nucleotides & Nucleic Acids, 19(1&2), 297-327 (2000)).

Wege für die Synthese von 2-Amino-4,6-dichlor-5-formamidopyrimidin sind entsprechend dem Stand der Technik bereits beschrieben. Den bekannten Verfahren liegt das Prinzip zugrunde, dass 2,5-Diamino-4,6-dihydroxypyrimidin (bzw. ein Salz desselben) mit einem chlorierenden Agens und einem Formamid und/oder einem Reagens vom Vilsmeyer-Typ umgesetzt wird. Dabei werden die in ungeschützter Form zu Nebenreaktionen neigenden Aminogruppen als Formamidine geschützt, die Hydroxygruppen chloriert und in nachfolgenden Schritten die Schutzgruppen partiell oder vollständig wieder abgespalten. Die gesamte Synthesesequenz lässt sich mit folgendem Schema verdeutlichen:

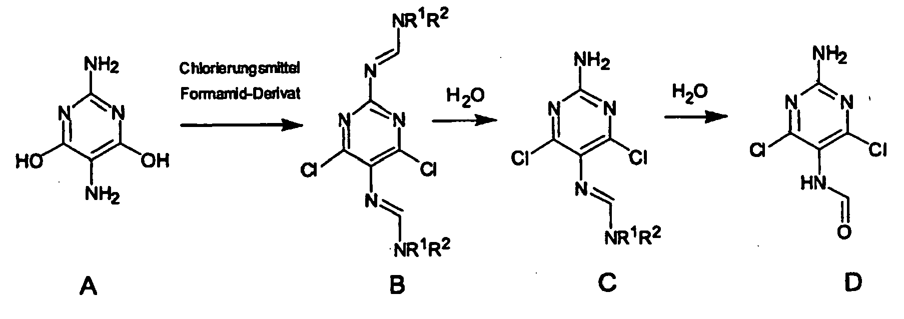

Diese Reaktion wird in der Patentliteratur bereits ausführlich behandelt. Beispielsweise wird gemäß US 6,552,193 die Umsetzung von 2,5-Diamino-4,6-dihydroxpyrimidin-Hemisulfat A mit Chloromethylen-dimethylammoniumchlorid (Vilsmeyer-Reagens) in Chloroform zu B (R¹, R²= CH₃) in 81 %iger Ausbeute, dessen Hydrolyse zu C in 95 %iger Ausbeute sowie die Weiterreaktion von C zu 2-Amino-4,6-dichlor-5-formamidopyrimidin D in einem Phosphat-Puffer mit 68 % Ausbeute beschrieben. Die Gesamt-Ausbeute über alle 3 Stufen beträgt 52 %. Gemäß der Lehre aus US 6,552,193 ist für den ersten Reaktionsschritt (die Chlorierung) ein inertes Lösemittel, z. B. Dichlormethan, Chloroform oder Dichlorethan erforderlich.

US 5,663,340 bzw. EP 684 236 beschreiben die Chlorierung von A mit Phosphoroxychlorid in Gegenwart von Dimethylformamid unter Verwendung eines inerten Lösemittels (beispielhaft genannt sind Toluol, Xylol, Chloroform, Dichlormethan, Dichlorethan, Chlorbenzol) unter Bildung von B (R¹, R² = CH₃) und Weiterreaktion zu C (ohne Isolierung von B) in 85 %iger Ausbeute. Die Umsetzung von C zu D erfolgt in Gegenwart von wässriger Propionsäure in 64 %iger Ausbeute. Die Gesamt-Ausbeute beträgt somit 54%.

Diese genannten Verfahren gemäß dem Stand der Technik erfüllen zwar ihren Zweck, 2-Amino-4,6-dichlor-5-formamidopyrimidi D als Zwischenprodukt zur Herstellung antiviraler Pharmazeutika zur Verfügung zu stellen, offenbaren jedoch deutliche Nachteile. Für den Chlorierungsschritt a) werden erhebliche Mengen an chlorierten und/oder aromatischen Lösemitteln verwendet. Dies ergibt eine ungünstige Raum-Zeit-Ausbeute sowie eine erhebliche Umweltbelastung. Die aus dem Chlorierungsschritt erhaltenen salzhaltigen Abwässer müssen entsorgt werden, für den abschließenden Hydrolyseschritt von C zu D muss jedoch erneut ein Reagens (Phosphat-Puffer bzw. Propionsäure) eingesetzt werden.

Zudem ist aus dem Stand der Technik - trotz einiger Versuche mit "Eintopf-Varianten"- nicht ersichtlich, dass ein direktes Verfahren zur Herstellung von D aus A ohne Isolierung der Zwischenprodukte möglich sein könnte. All diese Faktoren erhöhen die Mengen der eingesetzten Rohstoffe und der zu entsorgenden Reststoffe, verschlechtern die Raum-Zeit-Ausbeute in der Produktion und führen zusätzlich zu einer erheblichen Umweltbelastung.

Der Erfindung lag daher die Aufgabe zugrunde, ein einfaches und umweltfreundliches Verfahren zur Herstellung von 2-Amino-4,6-dichlor-5-formamidopyrimidin aus 2,5-Diamino-4,6-dihydroxypyrimidin oder einem Salz desselben mit hohen Raum-Zeit-Ausbeuten zu entwickeln. Zusätzlich sollte das Verfahren so vereinfacht werden, dass auf die Isolierung von Zwischenprodukten verzichtet werden kann (Eintopf-Reaktion).

Die Aufgabe der Erfindung wurde dadurch gelöst, dass man
a) das 2,5-Diamino-4,6-dihydroxypyrimidin bzw. dessen Salz oder tautomere Formen mit einem Chlorierungsmittel und einem Formamid der Formel (I) 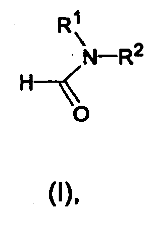 wobei
   R¹ und R² unabhängig einen C₁-C₄-Alkylrest bedeuten oder -R¹-R²- für - (CH₂)ₙ- mit n = 4 bis 6 oder -(CH₂)₂-O-(CH₂)₂- stehen, ohne Zusatz eines Lösemittels bei 50 bis 130 °C umsetzt,
b) das Reaktionprodukt aus Stufe a) bei 0 bis 100 °C mit Wasser umsetzt sowie mit einer anorganischen Base auf einen pH-Wert von 1,0 bis 6,0 einstellt und
c) die wässrige Reaktionsmischung aus Stufe b) bei 70 bis 120 °C unter Hydrolyse zu 2-Amino-4,6-dichlor-5-formamidopyrimidin reagieren lässt.

Überraschenderweise wurde nämlich gefunden, dass bei geeigneter Reaktionsführung die Reaktionsmischung im Chlorierungsschritt ohne Lösemittel besser rührbar ist als unter Verwendung eines Lösemittels, und dass aufgrund der Löslichkeitsverhältnisse von Produkt und Nebenprodukten 2-Amino-4,6-dichlor-5-formamidopyrimdin aus dem komplexen Reaktionsgemisch in hoher Reinheit erhalten werden kann.

Als Rohstoff für das erfindungsgemäße Verfahren wird 2,5-Diamino-4,6-dihydroxypyrimidin bzw. dessen Salz oder tautomere Formen eingesetzt. Wegen der leichten Oxidierbarkeit der freien Base sind insbesondere das Hemisulfat, das Hydrochlorid-Monohydrat und das wasserfreie Hydrochlorid besonders geeignet. Um unnötigen Reagensverbrauch bzw. unerwünschte Sulfat-Ionen zu vermeiden, wird wasserfreies 2,5-Diamino-4,6-dihydroxypyrimidin-Hydrochlorid besonders bevorzugt verwendet.

Als Chlorierungsmittel können verschiedene anorganische und organische Reagenzien mit der Funktionalität eines Säurechlorids eingesetzt werden. Beispielhaft seien Phosgen, Oxalylchlorid, Chloromethylen-dimethylammoniumchlorid (Vilsmeyer-Reagens), Thionylchlorid, Sulfurylchlorid, Phosphortrichlorid, Phosphorpentachlorid und Phosphoroxychlorid genannt. Als besonders bevorzugt wird Phosphoroxychlorid verwendet.

Das Formamid gemäß Formel (I) dient dazu, die Aminogruppen des Ausgangsstoffs zu formylieren und als Formamidin zu schützen.

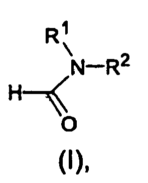

Als Zwischenprodukte werden in Stufe a) die 2,5-Diformamidino-4,6-dichlorpyrimidine der Formel (II) erhalten:

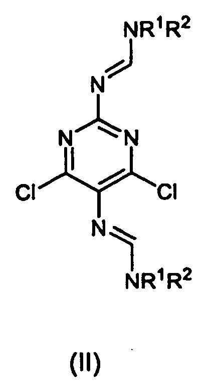

Die jeweiligen Reste R¹ und R² werden bei der weiteren Reaktion zum Endprodukt wieder abgespalten, so dass unabhängig vom eingesetzten Amid der Formel I immer dasselbe Endprodukt erhalten wird. Die Reste R¹ und R² bedeuten unabhängig voneinander einen C, bis C₄-Alkylrest und insbesondere Methyl, Ethyl, n-Propyl oder/und n-Butyl. Alternativ können die Reste R¹ und R² über eine Einfachbindung verknüpft sein und die Bedeutung -(CH₂)ₙ- mit n = 4 bis 6 oder -(CH₂)₂-O-(CH₂)₂- annehmen. Bevorzugte Amide der Formel (I) sind N,N-Dimethylformamid, N-Formylpyrrolidin, N-Formylpiperidin und N-Formylmorpholin. Besonders bevorzugt ist N,N-Dimethylformamid.

Die molaren Verhältnisse der Reaktanden im Chlorierungsschritt können in weiten Grenzen variiert werden. Bevorzugt werden pro 1 mol 2,5-Diamino-4,6-dihydroxypyrimidin 1 bis 5 mol Formamid der Formel (I) eingesetzt. Weiterhin bevorzugt werden 3 bis 7 mol Chlorierungsmittel pro 1 mol 2,5-Diamino-4,6-dihydroxypyrimidin eingesetzt. Für den Spezialfall der Verwendung von Phosphoroxychlorid und N,N-Dimethylformamid werden bevorzugt 3 bis 5 mol Phosphoroxychlorid und 1 bis 3 mol N,N-Dimethylformamid pro mol 2,5-Diamino-4,6-dihydroxypyrimidin verwendet.

Gemäß einer bevorzugten Ausführungsform wird zuerst das Chlorierungsmittel mit dem Formamid gemischt und erst in einem zweiten Schritt das 2,5-Diamino-4,6-dihydroxypyrimidin gegebenenfalls langsam dosiert bzw. portionsweise zugesetzt. Auf diese Weise wird erreicht, dass das zugesetzte unlösliche 2,5-Diamino-4,6-dihydroxypyrimidin laufend zu löslichen Folgeprodukten der Struktur (II) abreagiert, so dass die Rührbarkeit gewährleistet bleibt.

In einer bevorzugten Ausführungsform wird das Chlorierungsmittel vorgelegt. Dann gibt man bei einer Temperatur von 20 bis 100 °C, bevorzugt 40 bis 70 °C das N,N-Dialkylformamid zu und lässt das Reaktionsgemisch für eine Dauer von 5 bis 180 Minuten bei dieser Temperatur reagieren. Das 2,5-Diamino-4,6-dihydroxypyrimidin wird bei einer Temperatur von 50 bis 130 °C, bevorzugt 50 bis 100 °C im Verlauf von 15 Minuten bis 5 Stunden zudosiert. Anschließend erfolgt eine Nachreaktion während 1 bis 30 Stunden bei einer Temperatur von 50 bis 130 °C, bevorzugt 70 bis 110 °C.

In einer bevorzugten Ausführungsform erfolgt der Reaktionsschritt a) in einem Temperaturbereich von 70 bis 110 °C.

Der nachfolgende Hydrolyseschritt kann prinzipiell auf zwei verschiedene Weisen durchgeführt werden. Entweder wird die erforderliche Wassermenge direkt zu der Chlorierungsmischung dosiert. Dies ist vorteilhaft, da kein weiterer Reaktionsbehälter erforderlich ist, hat jedoch den Nachteil einer aufgrund der hohen Wärmeproduktion längeren Dosierzeit. Alternativ kann mit demselben Ergebnis die Chlorierungsmischung zu vorgelegtem Wasser dosiert werden.

Das zugesetzte bzw. vorgelegte Wasser sollte ausreichen, nach Ende der Hydrolyse eine gut rührbare Reaktionsmischung zu erhalten. Erfahrungsgemäß reichen hierfür 2 bis 5 Liter Wasser pro 1 mol eingesetztes 2,5-Diamino-4,6-dihydroxypyrimidin aus.

Der Hydrolyseschritt b) sollte im Temperaturbereich von 0 bis 100 °C erfolgen. Bevorzugt ist der Bereich von 20 bis 60 °C anzusehen.

Anschließend wird die erhaltene Reaktionsmischung mit einer anorganischen Base auf einen bestimmten pH-Wert eingestellt und auf diese Weise partiell hydrolysiert.

Als anorganische Basen sind prinzipiell alle Basen geeignet, die lösliche Chlorid-Salze bilden. Bevorzugt sind Natronlauge, Natriumhydroxid, Natriumcarbonat, Natriumhydrogencarbonat, Kalilauge, Kaliumhydroxid, Kaliumcarbonat, Kaliumhydrogencarbonat. Besonders bevorzugt ist Natronlauge. Die Menge der zugesetzten Base richtet sich nach dem einzustellenden pH-Wert und beträgt typischerweise 2 bis 3 mol pro mol eingesetztem Chlorierungsmittel.

Der pH-Wert ist von entscheidender Bedeutung, da er die selektive Reaktion von B über C zu D steuert. Bei falsch gewähltem pH-Wert werden eine reduzierte Ausbeute und/oder unerwünschte Nebenprodukte im Produkt erhalten. Erfindungsgemäß wird der pH auf einen definierten Wert im Bereich zwischen pH 1,0 und 6,0, bevorzugt pH 2,0 bis 5,0, besonders bevorzugt 3,0 bis 4,0 eingestellt, wobei der pH mittels einer Glaselektrode bei einer Temperatur von 20 °C gemessen wird. Gegebenenfalls kann der pH-Wert im Verlaufe der nachfolgenden Reaktion laufend nachgeregelt werden, indem pH-gesteuert weitere Base zugegeben wird.

Die weitere Reaktion wird durch Erhitzen der wässrigen Mischung auf eine Temperatur von 70 bis 120 °C, bevorzugt 80 bis 100 °C durchgeführt. Im Verlauf einer Reaktionszeit von 1 bis 20 Stunden bildet sich aus den nicht isolierten Zwischenprodukten das gewünschte Zielprodukt 2-Amino-4,6-dichlor-5-formamidopyrimidin. Dieses ist in der Reaktionsmischung unlöslich und kann mittels dem Fachmann geläufigen Prozessschritten abgetrennt, gewaschen und getrocknet werden.

Es ist als erfindungswesentlich anzusehen, dass dieser letzte Reaktionsschritt in Abwesenheit - auch von Spuren - eines Lösemittels erfolgt. Es hat sich nämlich gezeigt, dass dieses das in Wasser unlösliche 2-Amino-4,6-dichlor-5-formamidopyrimidin im Reaktionsgemisch anzulösen vermag, dadurch wird das Pyrimidin für eine weitere Hydrolyse angreifbarer, so dass letztlich reduzierte Ausbeuten und/oder eine Verunreinigung des Produkts mit 2,5-Diamino-4,6-dichlorpyrimidin, dem Folgeprodukt der Hydrolyse, resultieren.

Das erfindungsgemäße Verfahren liefert zufriedenstellende Ausbeuten, die nur wenig unter den Ausbeuten der Verfahren gemäß dem Stande der Technik liegen. Dafür bietet es den Vorteil einer höheren Reinheit des Endprodukts. Aufgrund der erheblich reduzierten Reaktionsvolumina, der eingesparten Lösemittel, Hilfs- und Reststoffe sowie des prozesstechnisch erheblich vereinfachten Verfahrens ergeben sich deutlich günstigere Herstellkosten für 2-Amino-4,6-dichlor-5-formylaminopyrimidin.

Ein weiterer Aspekt der Erfindung betrifft die Verwendung des nach dem erfindungsgemäßen Verfahrens hergestellten 2-Amino-4,6-dichlor-5-formylaminopyrimidins zur Herstellung von Purinderivaten. Weiterhin betrifft die Erfindung die Verwendung des nach dem erfindungsgemäßen Verfahrens hergestellten 2-Amino-4,6-dichlor-5-formylaminopyrimidins zur Herstellung von pharmazeutischen Wirkstoffen, insbesondere für antivirale Medikamente, z. B. zur Behandlung von AIDS.

Die nachfolgenden Beispiele dienen zur Veranschaulichung des gefundenen Verfahrens, ohne die Erfindungsbreite einzuengen.

### Beispiele

### Beispiel 1

61,33 g (0,40 mol) Phosphoroxychlorid wurden vorgelegt. Bei 50 °C wurden innerhalb von 45 Minuten 18,27 g (0,25 mol) Dimethylformamid zugetropft. Dann wurde auf 70 °C aufgeheizt und innerhalb von 45 Minuten 17,86 g (0,10 mol) 2,5-Diamino-4,6-dihydroxypyrimidin-Hydrochlorid spatelweise zugesetzt. Anschließend wurde auf 90 °C aufgeheizt und 20 Stunden gerührt. Es bildete sich eine dunkle, mäßig viskose, jedoch homogene und gut rührbare Mischung. Es wurde auf 20 °C abgekühlt und unter äußerer Kühlung mit 200 g Wasser versetzt. Durch Zugabe von 82,03 g 50 %iger Natronlauge wurde der pH von - 0,6 auf 4,0 gestellt, das Reaktionsgemisch auf 90 °C aufgeheizt und 8 Stunden gerührt. Es wurde auf 18 °C abgekühlt, das ausgefallene Produkt abgesaugt, mit Wasser gewaschen und im Vakuum getrocknet.

Es wurden 8,28 g reines 2-Amino-4,6-dichlor-5-formamidopyrimidin mit einem Gehalt von 98,7 % erhalten. Die Ausbeute bezogen auf eingesetztes 2,5-Diamino-4,6-dihydroxypyrimidin betrug 39,5 %.

### Beispiel 2

61,33 g (0,40 mol) Phosphoroxychlorid wurden vorgelegt und auf 50 °C aufgeheizt. Innerhalb von 45 Minuten wurden 29,24 g (0,40 mol) Dimethylformamid zugetropft. Dann wurde auf 72 °C aufgeheizt und innerhalb von 45 Minuten 17,86 g (0,10 mol) 2,5-Diamino-4,6-dihydroxypyrimidin-Hydrochlorid zugesetzt. Es wurde auf 90 °C aufgeheizt und 17 Stunden gerührt. Dann wurde auf 20 °C abgekühlt und unter äußerer Kühlung mit 200 g Wasser versetzt. Durch Zugabe von 88,35 g 50 %iger Natronlauge wurde der pH von - 0,5 auf 3,6 gestellt, das Reaktionsgemisch auf 97 °C aufgeheizt und 4 Stunden gerührt. Es wurde auf 18 °C abgekühlt, das ausgefallene Produkt abgesaugt, mit Wasser gewaschen und im Vakuum getrocknet.

Es wurden 7,92 g reines 2-Amino-4,6-dichlor-5-formamidopyrimidin mit einem Gehalt von 97,6 % erhalten. Die Ausbeute bezogen auf eingesetztes 2,5-Diamino-4,6-dihydroxypyrimidin betrug 37,4 %.

### Beispiel 3 (Vergleich)

180 ml Toluol und 76,7 g (0,5 mol) Phosphoroxychlorid wurden vorgelegt. Bei 50 °C wurden innerhalb 45 Minuten 29,2 g Dimethylformamid zugetropft. Bei 70 °C wurden dann 17,86 g (0,1 mol) 2,5-Diamino-4,6-dihydroxypyrimidin-Hydrochlorid portionsweise zugegeben. Anschließend wurde 20 Stunden bei 90 °C gerührt. Es bildete sich eine zähe Masse, die an Rührer und Kolbenwand klebte, und nur partiell in Toluol löslich war.

Nach Abkühlung wurde zu der Mischung 300 g Wasser dosiert, durch Zugabe von 89,6 g Natronlauge 50 % der pH auf 5,0 gestellt und die Toluolphase abgetrennt. Es bildete sich eine schwer trennbare Grenzschicht. Nach Eindampfen der Toluolphase verblieben 15,9 g rohes 2,5-Bis-(dimethylaminomethylenamino)-4,6-dichlorpyrimidin.

Die Wasserphase wurde 3 mal mit je 200 ml Essigsäureethylester extrahiert, die organischen Phasen eingedampft. Es verblieben 9,8 g einer zweiten, unreineren Fraktion von 2,5-Bis-(dimethylaminomethylenamino)-4,6-dichlorpyrimidin.

250 g Wasser und 5,7 g 85 %ige Phosphorsäure wurden vorgelegt, mit 3,7 g Natronlauge auf pH 4,0 gestellt und die Mischung der beiden Rohprodukte zugegeben. Dann wurde mit 10,5 g Phosphorsäure abermals auf pH 4,0 gestellt. Die Mischung wurde 4 Stunden bei 100 °C gerührt. Nach Abkühlung wurde das ausgefallene Produkt abfiltriert, gewaschen und getrocknet. Es wurden 11,3 g 2-Amino-4,6-dichlor-5-formamidopyrimidin mit einem Gehalt von 83,8% erhalten. Die Reinausbeute bezogen auf eingesetztes 2,5-Diamino-4,6-dihydroxypyrimidin betrug 45,7 %.

Ein analog durchgeführter Versuch unter Verwendung von Chlorbenzol anstatt Toluol führte zu besserer Rührbarkeit der Reaktionsmischung. Nach 3-maliger Extraktion mit Chlorbenzol und analoger Reaktion im Phosphatpuffer wurden 12,4 g 2-Amino-4,6-dichlor-5-formamidopyrimidin mit einem Gehalt von 78,3 % erhalten. Es stellte sich heraus, dass unvollständig abdestilliertes Chlorbenzol eine partielle Weiterhydrolyse zu 2,5-Diamino-4,6-dichlorpyrimidin bewirkt hatte.

## Patentansprüche

1. Verfahren zur Herstellung von 2-Amino-4,6-dichlor-5-formamidopyrimidin aus 2,5-Diamino-4,6-dihydroxypyrimidin bzw. einem Salz desselben,
**dadurch gekennzeichnet,**
**dass**
a) das 2,5-Diamino-4,6-dihydroxypyrimidin bzw. dessen Salz oder tautomere Formen mit einem Chlorierungsmittel und einem Formamid der Formel (I) 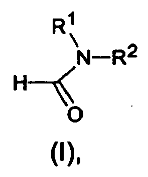 wobei
R¹ und R² unabhängig einen C₁-C₄-Alkylrest bedeuten oder -R¹-R²-für -(CH₂)ₙ- mit n = 4 bis 6 oder -(CH₂)₂-O-(CH₂)₂- stehen, ohne Zusatz eines Lösemittels bei 50 bis 130 °C umsetzt,
b) das Reaktionprodukt aus Stufe a) bei 0 bis 100 °C mit Wasser umsetzt sowie mit einer anorganischen Base auf einen pH-Wert von 1,0 bis 6,0 einstellt und
c) die wässrige Reaktionsmischung aus Stufe b) bei 70 bis 120 °C unter Hydrolyse zu 2-Amino-4,6-dichlor-5-formamidopyrimidin reagiert wird.

2. Verfahren nach Anspruch 1,
**dadurch gekennzeichnet,**
**dass** als Ausgangsprodukt 2,5-Diamino-4,6-dihydroxypyrimidin als Hemisulfat, Hydrochlorid-Monohydrat oder als wasserfreies Hydrochlorid, bevorzugt wasserfreies 2,5-Diamino-4,6-dihydroxypyrimidin-Hydrochlorid als Rohstoff eingesetzt wird.

3. Verfahren nach Anspruch 1 oder 2,
**dadurch gekennzeichnet,**
**dass** als Chlorierungsmittel ein Reagenz mit der Funktionalität eines Säurechlorids eingesetzt wird, bevorzugt Phosgen, Oxalylchlorid, Choromethylendimethylammoniumchlorid, Thionylchlorid, Sulfurylchlorid, Phosphortrichlorid, Phosphorpentachlorid oder Phosphoroxychlorid, besonders bevorzugt Phosphoroxychlorid.

4. Verfahren nach einem der Ansprüche 1 bis 3,
**dadurch gekennzeichnet,**
**dass** das Amid der Formel (I) in einem vorgelagerten Schritt mit dem Chlorierungsmittel umgesetzt und das 2,5-Diamino-4,6-dihydroxypyrimidin erst anschließend portionsweise zugegeben wird.

5. Verfahren nach einem der Ansprüche 1 bis 4,
**dadurch gekennzeichnet,**
**dass** N,N-Dimethylformamid , N-Formylpyrrolidin, N-Formylpiperidin oder N-Formylmorpholin, bevorzugt N,N-Dimethylformamid eingesetzt wird.

6. Verfahren nach einem der Ansprüche 1 bis 5,
**dadurch gekennzeichnet,**
**dass** 1,0 bis 5,0 mol Amid der Formel (I) pro mol 2,5-Diamino-4,8-dihydroxypyrimidin eingesetzt werden.

7. Verfahren nach einem der Ansprüche 1 bis 6,
**dadurch gekennzeichnet,**
**dass** 3,0 bis 7,0 mol Chlorierungsmittel pro mol 2,5-Diamino-4,6-dihydroxypyrimidin eingesetzt werden.

8. Verfahren nach einem der Ansprüche 1 bis 7,
**dadurch gekennzeichnet, dass** der Reaktionsschritt a) in einem Temperaturbereich von 70 bis 110 °C erfolgt.

9. Verfahren nach einem der Ansprüche 1 bis 8,
**dadurch gekennzeichnet,**
**dass** im Schritt b) als anorganische Base eine Base verwendet wird, welche lösliche Chloridsalze bildet, bevorzugt eine oder mehrere Verbindungen , welche aus der Gruppe Natronlauge, Natriumhydroxid, Natriumcarbonat, Natriumhydrogencarbonat, Kalilauge, Kaliumhydroxid, Kaliumcarbonat und Kaliumhydrogencarbonat ausgewählt werden .

10. Verfahren nach einem der Ansprüche 1 bis 9,
**dadurch gekennzeichnet,**
**dass** als Base Natronlauge verwendet wird.

11. Verfahren nach einem der Ansprüche 1 bis 10,
**dadurch gekennzeichnet,**
**dass** 2 bis 3 mol der anorganischen Base pro mol Chlorierungsmittel eingesetzt werden.

12. Verfahren nach einem der Ansprüche 1 bis 11,
**dadurch gekennzeichnet,**
**dass** im Schritt b) die partielle Neutralisation bis zu einem pH-Wert von 2,0 bis 5,0, vorzugsweise 3,0 bis 4,0, erfolgt.

13. Verfahren nach einem der Ansprüche 1 bis 12,
**dadurch gekennzeichnet,**
**dass** das Reaktionprodukt aus Stufe a) bei 20 bis 60 °C umgesetzt wird.

14. Verfahren nach einem der Ansprüche 1 bis 13,
**dadurch gekennzeichnet,**
**dass** die Hydrolyse im Schritt c) bei einer Temperatur von 70-120 °C, bevorzugt 80 bis 100 °C erfolgt.

15. Verfahren nach einem der Ansprüche 1 bis 13,
**dadurch gekennzeichnet,**
**dass** Schritt c) in Abwesenheit eines Lösemittels erfolgt.

16. Verfahren nach einem der Ansprüche 1 bis 15,
**dadurch gekennzeichnet,**
**dass** die beanspruchte Reaktion ohne Isolierung von Zwischenprodukten erfolgt, d.h. als Eintopfreaktion.

17. Verfahren zur Herstellung von Purinderivaten, umfassend die Verfahrensschritte nach einem der Ansprüche 1 bis 16 und weiterhin das Umsetzen von 2-Amino-4,6-dichlor-5-formamidopyrimidin zu einem Purinderivat.

18. Verfahren zur Herstellung von pharmazeutischen Wirkstoffen, umfassend die Verfahrensschritte nach Anspruch 17 und weiterhin das Umsetzen des Purinderivats zu einem pharmazeutischen Wirkstoff.

19. Verfahren nach Anspruch 18, worin der pharmazeutische Wirkstoff ein antivirales Medikament ist.

20. Verfahren nach Anspruch 19, worin das antivirale Medikament ein Medikament zur Behandlung von AIDS ist.

## Claims

1. Process for preparing 2-amino-4,6-dichloro-5-formamidopyrimidine from 2,5-diamino-4,6-dihydroxypyrimidine or a salt thereof,
**characterized in that**
a) the 2,5-diamino-4,6-dihydroxypyrimidine or salt or tautomeric forms thereof is reacted with a chlorinating agent and a formamide of the formula (I) 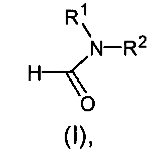 where
R¹ and R² are each independently a C₁-C₄-alkyl radical, or -R¹-R²- is -(CH₂)ₙ- where n = from 4 to 6 or -(CH₂)₂-O-(CH₂)2-, without addition of a solvent at from 50 to 130°C,
b) the reaction product from stage a) is reacted at from 0 to 100°C with water and adjusted to a pH of from 1.0 to 6.0 with an inorganic base and
c) the aqueous reaction mixture from stage b) is reacted at from 70 to 120°C with hydrolysis to give 2-amino-4,6-dichloro-5-formamidopyrimidine.

2. Process according to Claim 1,
**characterized in that**
the starting material used is 2,5-diamino-4,6-dihydroxypyrimidine as the hemisulfate, hydrochloride monohydrate or as the anhydrous hydrochloride, preferably anhydrous 2,5-diamino-4,6-dihydroxypyrimidine hydrochloride as the raw material.

3. Process according to Claim 1 or 2,
**characterized in that**
the chlorinating agent used is a reagent having the functionality of an acid chloride, preferably phosgene, oxalyl chloride, chloromethylenedimethylammonium chloride, thionyl chloride, sulfuryl chloride, phosphorus trichloride, phosphorus pentachloride or phosphorus oxychloride, more preferably phosphorus oxychloride.

4. Process according to one of Claims 1 to 3,
**characterized in that**
the amide of the formula (I) is reacted with the chlorinating agent in a preceding step and the 2,5-diamino-4,6-dihydroxypyrimidine is only then added in portions.

5. Process according to one of Claims 1 to 4,
**characterized in that**
N,N-dimethylformamide, N-formylpyrrolidine, N-formylpiperidine or N-formylmorpholine, preferably N,N-dimethylformamide, is used.

6. Process according to one of Claims 1 to 5,
**characterized in that**
from 1.0 to 5.0 mol of amide of the formula (I) per mole of 2,5-diamino-4,6-dihydroxypyrimidine are used.

7. Process according to one of Claims 1 to 6,
**characterized in that**
from 3.0 to 7.0 mol of chlorinating agent per mole of 2,5-diamino-4,6-dihydroxypyrimidine are used.

8. Process according to one of Claims 1 to 7,
**characterized in that**
reaction step a) is effected within a temperature range from 70 to 110°C.

9. Process according to one of Claims 1 to 8,
**characterized in that**
the inorganic base used in step b) is a base which forms soluble chloride salts, preferably one or more compounds which are selected from the group of sodium hydroxide solution, sodium hydroxide, sodium carbonate, sodium hydrogencarbonate, potassium hydroxide solution, potassium hydroxide, potassium carbonate and potassium hydrogencarbonate.

10. Process according to one of Claims 1 to 9,
**characterized in that**
the base used is sodium hydroxide solution.

11. Process according to one of Claims 1 to 10,
**characterized in that**
from 2 to 3 mol of the inorganic base are used per mole of chlorinating agent.

12. Process according to one of Claims 1 to 11,
**characterized in that**
the partial neutralization in step b) is effected up to a pH of from 2.0 to 5.0, preferably from 3.0 to 4.0.

13. Process according to one of Claims 1 to 12,
**characterized in that**
the reaction product from stage a) is reacted at from 20 to 60°C.

14. Process according to one of Claims 1 to 13,
**characterized in that**
the hydrolysis in step c) is effected at a temperature of 70-120°C, preferably from 80 to 100°C.

15. Process according to one of Claims 1 to 13,
**characterized in that**
step c) is effected in the absence of a solvent.

16. Process according to one of Claims 1 to 15,
**characterized in that**
the claimed reaction is effected without isolation of intermediates, i.e. as a one-pot reaction.

17. Process for preparing purine derivatives, comprising the process steps according to one of Claims 1 to 16 and also the conversion of 2-amino-4,6-dichloro-5-formamidopyrimidine to a purine derivative.

18. Process for preparing active pharmaceutical ingredients, comprising the process steps according to Claim 17 and also the conversion of the purine derivative to an active pharmaceutical ingredient.

19. Process according to Claim 18, wherein the active pharmaceutical ingredient is an antiviral medicament.

20. Process according to Claim 19, wherein the antiviral medicament is a medicament for the treatment of AIDS.

## Revendications

1. Procédé pour la préparation de 2-amino-4,6-dichloro-5-formamidopyrimidine à partir de 2,5-diamino-4,6-dihydroxypyrimidine ou d'un sel de celle-ci, **caractérisé en ce que**
a) on fait réagir à 50-130 °C, sans addition d'un solvant, la 2,5-diamino-4,6-dihydroxypyrimidine ou un sel ou des formes tautomères de celle-ci avec un agent de chloration et un formamide de formule (I) 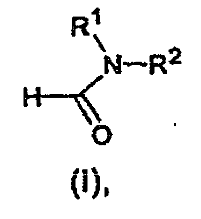 dans laquelle
R¹ et R² représentent indépendamment un radical alkyle en C₁-C₄ ou bien -R¹-R²-représentent - (CH₂)ₙ- où n = 4 à 6 ou -(CH₂)₂-O-(CH₂)₂-,
b) on fait réagir avec de l'eau, à 0-100 °C, le produit de réaction provenant de l'étape a) et on ajuste à un pH de 1,0 à 6,0 à l'aide d'une base minérale et
c) on fait réagir le mélange réactionnel provenant de l'étape b), à 70-120 °C, par hydrolyse, pour obtenir la 2-amino-4,6-dichloro-5-formamidopyrimidine.

2. Procédé selon la revendication 1, **caractérisé en ce qu'**on utilise comme produit de départ de la 2,5-diamino-4,6-dihydroxypyrimidine sous forme d'hémisulfate, de chlorhydrate-monohydrate ou de chlorhydrate anhydre, de préférence le chlorhydrate de 2,5-diamino-4,6-dihydroxypyrimidine anhydre en tant que matière première.

3. Procédé selon la revendication 1 ou 2, **caractérisé en ce qu'**on utilise comme agent de chloration un réactif ayant la fonctionnalité d'un chlorure d'acide, de préférence le phosgène, le chlorure d'oxalyle, le chlorure de chlorométhylènediméthylammonium, le chlorure de thionyle, le chlorure de sulfuryle, le trichlorure de phosphore, le pentachlorure de phosphore ou l'oxychlorure de phosphore, de façon particulièrement préférée l'oxychlorure de phosphore.

4. Procédé selon l'une quelconque des revendications 1 à 3, **caractérisé en ce qu'**on fait réagir l'amide de formule (I), dans une étape préliminaire, avec l'agent de chloration et seulement ensuite on ajoute par portions la 2,5-diamino-4,6-dihydroxypyrimidine.

5. Procédé selon l'une quelconque des revendications 1 à 4, **caractérisé en ce qu'**on utilise le N,N-diméthylformamide, la N-formylpyrrolidine, la N-formylpipéridine ou la N-formylmorpholine, de préférence le N,N-diméthylformamide.

6. Procédé selon l'une quelconque des revendications 1 à 5, **caractérisé en ce qu'**on utilise 1,0 à 5,0 moles de l'amide de formule (I) par mole de 2,5-diamino-4,6-dihydroxypyrimidine.

7. Procédé selon l'une quelconque des revendications 1 à 6, **caractérisé en ce qu'**on utilise 3,0 à 7,0 moles d'agent de chloration par mole de 2,5-diamino-4,6-dihydroxypyrimidine.

8. Procédé selon l'une quelconque des revendications 1 à 7, **caractérisé en ce que** l'étape de réaction a) s'effectue dans une plage de température de 70 à 110 °C.

9. Procédé selon l'une quelconque des revendications 1 à 8, **caractérisé en ce qu'**on utilise comme base minérale dans l'étape b) une base qui forme des chlorures solubles, de préférence un ou plusieurs composés qui sont choisis dans le groupe constitué par la lessive de soude, l'hydroxyde de sodium, le carbonate de sodium, l'hydrogénocarbonate de sodium, la lessive de potasse, l'hydroxyde de potassium, le carbonate de potassium et l'hydrogénocarbonate de potassium.

10. Procédé selon l'une quelconque des revendications 1 à 9, **caractérisé en ce qu'**on utilise comme base la lessive de soude.

11. Procédé selon l'une quelconque des revendications 1 à 10, **caractérisé en ce qu'**on utilise 2 à 3 moles de la base minérale par mole d'agent de chloration.

12. Procédé selon l'une quelconque des revendications 1 à 11, **caractérisé en ce que** dans l'étape b) la neutralisation partielle s'effectue jusqu'à un pH de 2,0 à 5,0, de préférence de 3,0 à 4,0.

13. Procédé selon l'une quelconque des revendications 1 à 12, **caractérisé en ce que** le produit de réaction provenant de l'étape a) est mis en réaction à 20-60 °C.

14. Procédé selon l'une quelconque des revendications 1 à 13, **caractérisé en ce que** l'hydrolyse dans l'étape c) s'effectue à une température de 70 à 120 °C, de préférence de 80 à 100 °C.

15. Procédé selon l'une quelconque des revendications 1 à 13, **caractérisé en ce que** l'étape c) s'effectue en absence d'un solvant.

16. Procédé selon l'une quelconque des revendications 1 à 15, **caractérisé en ce que** la réaction revendiquée s'effectue sans isolement de produits intermédiaires, c'est-à-dire en tant que réaction en un seul récipient.

17. Procédé pour la préparation de dérivés de purines, comprenant les étapes de processus selon l'une quelconque des revendications 1 à 16 et en outre la conversion de la 2-amino-4,6-dicloro-5-formamidopyrimidine en un dérivé de purine.

18. Procédé pour la préparation de substances actives pharmaceutiques, comprenant les étapes de processus selon la revendication 17 et en outre la conversion du dérivé de purine en une substance active pharmaceutique.

19. Procédé selon la revendication 18, dans lequel la substance active pharmaceutique est un médicament antiviral.

20. Procédé selon revendication 19, dans lequel le médicament antiviral est un médicament pour le traitement du SIDA.
